# FASCICULE DE BREVET EUROPEEN

(11) **EP 2 218 439 B1**
(45) Date de publication et mention de la délivrance du brevet: **15.06.2016**
(21) Numéro de dépôt: 10151790.2
(22) Date de dépôt: 27.01.2010
(51) Int. Cl.: A61K 8/37, A61K 8/92, A61Q 1/14

(54) **Gel démaquillant comprenant de l'huile de jojoba**
Jojobaöl enthaltendes Abschminkgel
Cleansing gel comprising Jojoba oil

(30) Priorité: 13.02.2009 FR 0950931
(43) Date de publication de la demande: 18.08.2010
(73) Titulaire: L'Oréal, 75008 Paris (FR)
(72) Inventeur: Poletti, Mickaël, 75012, PARIS (FR)
(74) Mandataire: Duvert, Sandra

(56) Documents cités:
- EP-A- 1 103 250
- JP-A- 5 229 916
- US-A- 5 603 940
- ANNA CRISTOFARO: "Uses of Jojoba Oil" , [Online] 21 février 2007 (2007-02-21), XP002556995 Extrait de l'Internet: URL:http://beauty-treatments.suite101.com/ article.cfm/uses_of_jojoba_oil> [extrait le 2009-11-23]
- GREEN HERBAL REMEDIES: "Jojoba Oil For Complete Winter Moisturising Needs" , [Online] 15 décembre 2008 (2008-12-15), XP002557192 Extrait de l'Internet: URL:http://www.greenherbalremedies.com/blo g/jojoba-oil-for-complete-winter-moisturis ing-needs/> [extrait le 2009-11-23]
- DATABASE CAPLUS [Online] CHEMICAL ABSTRACTS SERVICE, COLUMBUS, OHIO, US; 22 juin 1985 (1985-06-22), "Transparent cleansing gel compositions for the skin" XP002556997 extrait de STN Database accession no. 1985:600703 & JP 60 115509 A (SUNSTAR INC.) 22 juin 1985 (1985-06-22)

## Description

L'invention a pour objet une composition cosmétique comprenant de l'huile de jojoba et un émulsionnant ester de sucrose et d'acide gras et son utilisation dans le domaine cosmétique, notamment pour le démaquillage et/ou le nettoyage des matières kératiniques.

Les technologies du maquillage actuelles sont de plus en plus innovantes et performantes, et les utilisatrices de produits de maquillage sont de plus en plus nombreuses à utiliser des produits longue tenue tels que des fonds de teint non transferts, des rouges à lèvres de longue tenue, des mascaras waterproof ou des mascaras double geste (application d'une base puis d'un top coat de mascara). Toutefois, ce type de produits est plus difficile à enlever que les produits classiques de maquillage, et il existe, de ce fait, un besoin de produits démaquillants qui soient à la fois très performants, pratiques à utiliser et qui assurent un respect de la peau tout en ayant de bonnes qualités cosmétiques (bon confort d'utilisation, douceur).

En outre, depuis quelques années le marché cosmétique est marqué par une demande très forte de formulations contenant des ingrédients d'origine naturelle. Les consommateurs désirent des formulations exemptes de matières chimiques auxquels ils préfèrent des ingrédients d'origine naturelle, réputés pour leur meilleure tolérance et affinité avec la peau, et qui soient plus respectueuses de l'environnement.

Il subsiste donc le besoin de disposer d'un produit de démaquillage comprenant des ingrédients d'origine naturelle présentant une bonne innocuité vis-à-vis des matières kératiniques, tout en ayant néanmoins les propriétés requises pour des produits à la fois performant sur tous types de maquillages (classiques et longue tenue), pratique à utiliser et confortable.

La demanderesse a découvert que l'association d'un tensioactif ester de sucrose et d'acide gras avec une huile végétale particulière, en une teneur déterminée, permet d'atteindre ces objectifs.

La présente invention a donc pour objet une composition cosmétique comprenant :
- une phase aqueuse comprenant éventuellement un ou plusieurs polyols, la quantité de polyol(s) allant alors de 0,5 à 15 % en poids par rapport au poids total de la composition,
- au moins 35 % en poids d'huile de jojoba par rapport au poids total de la composition, et
- au moins un ester de sucrose et d'acide gras.

La composition selon l'invention présente l'avantage d'être gélifiée, donc facile à utiliser, tant en étant confortable et en permettant un bon démaquillage de tout type de maquillage, en particulier sur les maquillages waterproof ou longue tenue. Elle présente en outre une bonne stabilité.

La composition selon l'invention se présente de préférence sous forme gélifiée, en particulier sous forme d'un gel de type émulsion huile dans eau, comprenant une phase grasse dispersée dans une phase aqueuse. On entend par « gélifiée » le fait que la composition ne coule pas, c'est-à-dire qu'elle ait une certaine viscosité. Sa viscosité peut aller par exemple de 5 à 190 poises (0,5 à 19 Pa.s), de préférence de 5 à 150 poises (0,5 à 15 Pa.s) et mieux de 10 à 120 poises (1 à 12 Pa.s), viscosité mesurée au viscosimètre RHEOMAT 180 à un taux de cisaillement de 200 s⁻¹ et à 25 °C.

La composition selon l'invention est destinée à une application topique et contient donc un milieu physiologiquement acceptable. On entend ici par « milieu physiologiquement acceptable » un milieu compatible avec les matières kératiniques telles que la peau, les muqueuses, le cuir chevelu, les yeux et/ou les fibres kératiniques telles que les cils ou les cheveux.

### Ester de sucrose et d'acide gras

Selon la présente invention, l'ester de sucrose et d'acide gras est de préférence choisi parmi les esters issus de la réaction de sucrose(s) (saccharose) et d'acide(s) gras comprenant de 10 à 24 atomes de carbone, de préférence de 12 à 20 atomes de carbone, mieux de 12 à 18 atomes de carbone et encore mieux de 12 à 16 atomes de carbone.
Les acides gras comprenant de 10 à 24 atomes de carbone peuvent être linéaires ou ramifiés, saturés ou insaturés.
Les acides gras peuvent être choisis parmi l'acide oléique, l'acide laurique, l'acide palmitique, l'acide myristique, l'acide stéarique, l'acide linoléique, l'acide caprique, ou leurs mélanges

Selon un mode de réalisation, l'ester de sucrose et d'acide gras est choisi parmi les esters issus de la réaction de sucrose et d'acide gras comprenant de 12 à 18 atomes de carbone, de préférence de 12 à 16 atomes de carbone tels que l'acide laurique et/ou l'acide palmitique comme par exemple le sucrose laurate, le sucrose palmitate ou un mélange.

Les esters de sucrose et d'acides gras peuvent être choisis parmi les mono-, di-, tri- et tétra-esters, les polyesters et leurs mélanges. On utilise de préférence des esters à faible degré d'estérification comme par exemple des monoesters, diesters, triesters de sucrose et d'acide gras ou un mélange. L'ester de sucrose et d'acide gras peut se présenter sous forme d'un mélange d'esters à faible degré d'estérification comme par exemple un mélange de monoester et diester ou un mélange de monoester, diester et triester.

Dans le cas ou l'on utilise un mélange d'esters de sucrose et d'acide gras, on préfère un mélange dans lesquel les esters à faible degré d'estérification, en particulier les monoesters, sont majoritaires et représentent par exemple au moins 50%, de préférence au moins 60% en poids du mélange d'esters de sucrose et d'acide gras.
On peut utiliser en particulier un mélange d'esters de sucrose et d'acides gras comprenant de 12 à 16 atomes de carbone, en particulier une mélange de mono, di et triesters d'acide laurique ou d'acide palmitique, ledit mélange pouvant comprendre de façon minoritaire (en une teneur inférieure ou égale à 40% en poids par rapport au poids du mélange d'esters de sucrose et d'acide gras) des esters de sucrose et d'acides gras dans lequel l'acide gras comprend plus de 16 atomes de carbone.

De préférence, l'ester de sucrose et d'acide gras utilisé dans la présente invention présente une HLB supérieure ou égale à 10, de préférence supérieure ou égale à 12. Comme cela est bien connu, on entend par HLB (Hydrophilic-Lipophilic Balance) l'équilibre entre la dimension et la force du groupe hydrophile et la dimension et la force de groupe lipophile de l'agent tensioactif.
La valeur HLB selon GRIFFIN est définie dans J. Soc. Cosm. Chem. 1954 (volume 5), pages 249-256.

On peut citer à titre d'exemples d'esters ou de mélanges d'esters de sucrose et d'acide gras :
- le Surfhope SE COSME C-1416, présentant une HLB de 16, qui est un sucrose myristate comprenant environ 80% de monoester, le reste du mélange étant composé de di et triesters,
- le Surfhope SE COSME C-1216 dont le nom INCI est sucrose laurate, de HLB égale à 16 et comprend de 75 à 90% de monoester, le reste du mélange étant composé de di et triesters,
- le Surfhope SE COSME C-1215L dont le nom INCI est sucrose laurate, de HLB HLB égale à 15, comprenant environ 70% de monoesters, le reste du mélange étant composé de diesters et autres polyetsers,
- le Surfhope SE COSME C-1616, présentant une HLB de 16, qui est un mélange d'esters de saccharose et d'acides palmitique et/ou stéarique (nom INCI sucrose palmitate), comprenant de 75 à 90% de monoester, le reste du mélange étant composé de di et triesters, et pouvant comprendre du sucrose stéarate et sucrose palmitate stéarate.

On peut également citer l'ester portent le nom INCI sucrose laurate commercialisé par la société Dai-ichi Seiyaku sous la référence DK ester S-L18A, de HLB égale à 17, comprenant 70% de monoesters et 30% de di- et tri-esters.

On peut aussi citer à titre d'exemples d'esters ou de mélanges d'esters de sucrose d'acide gras:
- les produits vendus sous les dénominations F160, F140, F110, F90, F70, SL40 par la société CRODESTA, désignant respectivement les palmito-stéarates de sucrose formés de 73% de monoester et 27% de di- et tri-ester, de 61% de monoester et 39% de di-, tri-, et tétra-ester, de 52% de monoester et 48% de di-, tri-, et tétra-ester, de 45% de monoester et 55% de di-, tri-, et tétra-ester, de 39% de monoester et 61% de di-, tri-, et tétra-ester, et le mono-laurate de sucrose;
- les produits vendus sous la dénomination RYOTO SUGAR ESTERS par exemple référencés B370 et correspondant au béhénate de saccharose formé de 20% de monoester et 80% de di-triester-polyester;
- le mono-di-palmito-stéarate de sucrose commercialisé par la société GOLDSCHMIDT sous la dénomination TEGOSOFT PSE.

La quantité d'ester(s) de sucrose et d'acide gras peut aller par exemple de 0,1 à 20 % en poids, de préférence de 0,5 à 15 % en poids, mieux de 1 à 15 % en poids, encore mieux de 2 à 10 % en poids par rapport au poids total de la composition.

Selon un mode de réalisation, la composition selon l'invention peut comprendre, outre l'ester de sucrose et d'acide gras, un tensioactif additionnel choisi parmi les tensioactifs anioniques, non ioniques ou amphotériques, en particulier des tensioactifs non ioniques, mais seulement dans la mesure où la présence de ces tensioactifs n'affecte pas le confort (innocuité) de la composition.
Les tensioactifs additionnels pouvant être utilisés dans la composition selon l'invention sont de préférence des tensioactifs d'origine naturelle, dans lesquels on inclut les composés qui peuvent être présents dans la nature et qui sont reproduits par synthèse chimique, ils peuvent être notamment choisis parmi les savons (sels d'acides gras), les dérivé de l'huile de soja, les dérivé d'acide lactique, les aminoacides, les acylaminoacides, leurs sels, les alkyl polyglucosides (APG), les gommes hydrophobées et leurs mélanges.

Selon un autre mode de réalisation, le ou les esters de sucrose et d'acide gras constituent le système tensioactif principal de la composition.
Par « système tensioactif principal », on entend un système qui, en son absence, ne conduit pas à la formation d'une composition stable.
Par « stable », on entend une composition qui, après avoir été placée dans une étuve à 45°C pendant deux mois, ne présente pas, après retour à température ambiante, de déphasage des phases grasse et aqueuse, ou de relargage de phase grasse en surface.

Selon un autre mode de réalisation, le ou les esters de sucrose et d'acide gras constituent l'unique système tensioactif de la composition..
Par « unique » on entend que tout éventuel système tensioactif additionnel est présent en une teneur n'excédant pas 1%, et de préférence n'excédant pas 0,5%. De préférence encore, par « unique » on désigne une absence totale de tout autre système tensioactif.

### Phase aqueuse

La composition selon l'invention comprend une phase aqueuse comprenant de l'eau et/ou des solvants hydrophiles comme des polyols.
L'eau est de préférence présente en une teneur inférieure ou égale à 20 % en poids par rapport au poids total de la composition, de préférence inférieure ou égale à 15% en poids et mieux inférieure ou égale à 10% en poids. La quantité d'eau dans la composition peut aller par exemple de 0,5 à 20 % en poids, de préférence de 0,6 à 15 % en poids, mieux de 0,6 à 13 % en poids, encore mieux de 1 à 10 % en poids et encore mieux de 3 à 10 % en poids par rapport au poids total de la composition.

L'eau utilisée dans la composition de l'invention peut être de l'eau pure déminéralisée mais aussi de l'eau minérale et/ou de l'eau thermale et/ou de l'eau de mer, c'est-à-dire que l'eau de la composition peut être en partie ou totalement constituée par une eau choisie parmi les eaux minérales, les eaux thermales, les eaux de mer et leurs mélanges. En général, une eau minérale est propre à la consommation, ce qui n'est pas toujours le cas d'une eau thermale. Chacune de ces eaux contient, entre autre, des minéraux solubilisés et/ou des oligo-éléments. Ces eaux sont connues pour être employées à des fins de traitement spécifique selon les oligo-éléments et les minéraux particuliers qu'elles contiennent, tel que l'hydratation et la désensibilisation de la peau ou le traitement de certaines dermatoses. Par eaux minérales ou thermales, on désignera non seulement les eaux minérales ou thermales naturelles, mais également des eaux minérales ou thermales naturelles enrichies en constituants minéraux et/ou en oligo-éléments supplémentaires, ainsi que des solutions aqueuses minérales et/ou contenant des oligo-éléments préparées à partir d'eau purifiée (déminéralisée ou distillée).

Une eau thermale ou minérale naturelle utilisée selon l'invention peut, par exemple, être choisie parmi l'eau de Vittel, les eaux du bassin de Vichy, l'eau d'Uriage, l'eau de la Roche Posay, l'eau de la Bourboule, l'eau d'Enghien-les-Bains, l'eau de Saint Gervais-les-Bains, l'eau de Néris-les-Bains, l'eau d'Allevar-les-Bains, l'eau de Digne, l'eau de Maizières, l'eau de Neyrac-les-Bains, l'eau de Lons-le-Saunier, les Eaux Bonnes, l'eau de Rochefort, l'eau de Saint Christau, l'eau des Fumades et l'eau de Tercis-les-bains, l'eau d'Avene.

La phase aqueuse de la composition de l'invention peut comprendre un ou plusieurs polyols. Parmi les polyols utilisables dans la composition selon l'invention, on peut citer notamment la glycérine, le propylène glycol, le butylène glycol, l'hexylène glycol, les polyéthylène glycols tels que le PEG-8, le dipropylène glycol et leurs mélanges. Selon un mode préféré de réalisation de l'invention, le polyol est la glycérine qui donne un meilleur confort à l'application. On peut ajouter à la glycérine, d'autres polyols dans la mesure où les qualités de la composition sont maintenues.

La quantité de polyol(s) peut aller par exemple de 0,5 à 15 % en poids, de préférence de 0,5 à 10 % en poids, mieux de 1 à 10 % en poids, encore mieux de 2 à 10 % en poids et encore mieux de 2 à 8 % en poids par rapport au poids total de la composition. La composition peut comprendre un gélifiant hydrophile, choisi de préférence parmi les gélifiants d'origine naturelle, en particulier d'origine végétale, ou les polysaccharides d'origine biotechnologique (par exemple la gomme de xanthane).
Ce polysaccharide issu du végétal peut le cas échéant être modifié chimiquement pour favoriser sa valence hydrophile, comme c'est le cas des dérivés de cellulose, en particulier des hydroxyalkyle celluloses (ex : hydroxyethylcellulose).

Comme exemples de polysaccharides d'origine végétale utilisables selon l'invention, on peut citer notamment :
a) des extraits d'algues, tels que les alginates, les carraghénanes, les agars agars, et leurs mélanges. A titre d'exemples de carraghénanes, on peut citer les Satiagum UTC30^{®} et UTC10^{®} de la société Degussa ; comme alginates, on peut citer l'alginate de sodium vendu sous la dénomination Kelcosol^{®} par la société ISP ;
b) des gommes, telles que la gomme de guar et leurs dérivés non ioniques (hydroxypropyl guar), la gomme arabique, la gomme de konjac ou mannane, la gomme Tragacanthe, la gomme Ghatti, la gomme Karaya, la gomme de caroube ; comme exemples, on peut citer la gomme de guar commercialisée sous la dénomination Jaguar HP105^{®} par la société Rhodia ; la gomme de mannane et konjac^{®} (1% de glucomannane) commercialisée par la société GfN ;
c) les amidons modifiés ou non, tels que ceux issus, par exemple, de céréales comme le blé, le maïs ou le riz, de légumes comme le pois blond, de tubercules comme les pommes de terre ou le manioc, les amidons de tapioca ; des dextrines, telles que les dextrines de mais ; comme exemples, on peut citer notamment l'amidon de riz Remy DR I^{®} commercialisé par la société Remy ; l'amidon de maïs B^{®} de la société Roquette ; la fécule de pomme de terre modifiée par l'acide 2-chloroethyl aminodipropionique neutralisé à la soude commercialisé sous la dénomination Structure Solanace^{®} par la société National Starch ; la poudre d'amidon de tapioca natif commercialisée sous la dénomination Tapioca pure^{®} par la société National Starch ;
d) les dextrines, telles que la dextrine extraite de maïs sous la dénomination Index^{®} de la société National Starch ;
e) les celluloses et leurs dérivés, en particulier les alkyle ou hydroxyalkyle celluloses ; on peut citer notamment les méthylcelluloses, hydroxyalkylcelluloses, éthylhydroxyéthylcelluloses, carboxyméthyl-celluloses. Comme exemples, on peut citer les cetyl hydroxy ethyl cellulose sous les dénominations Polysurf 67CS^{®} et Natrosol Plus 330^{®} d'Aqualon ;
et leurs mélanges.

Selon un mode de réalisation, la composition selon l'invention comprend moins de 1,5% en poids de polymères épaississants ou gélifiants synthétiques, de préférence moins de 1%, mieux moins de 0,5%, voire moins de 0,2% en poids. Elle peut être totalement exempte de polymères épaississants ou gélifiants synthétiques.
De tels polymères synthétiques sont par exemple des polymères acryliques (famille des Carbopol), des copolymères acryliques/alkyl acrylates ou des (co)polymères à base d'acide 2-acrylamido 2-méthylpropane sulfonique (par exemple les polymères commercialisés sous la dénomination Pemulen, Sepigel ou Simulgel, Aristoflex).

### Phase grasse

La phase grasse de la composition selon l'invention comprend l'ensemble des composés liposolubles ou lipodispersibles présents dans la composition, incluant les corps gras liquides à température ambiante (25°C) ou huiles (qui forment la phase huileuse), les corps gras solides à température ambiante tels que les cires, ou encore des composés pâteux, des alcools gras, des acides gras.

### Huile de jojoba

L'huile de jojoba est présente dans la composition en une teneur supérieure ou égale à 35% en poids, de préférence supérieure ou égale à 40 % en poids par rapport au poids total de la composition, de préférence supérieure ou égale à 50 % en poids, mieux supérieure ou égale à 60 % en poids et encore mieux supérieure ou égale 70 % en poids par rapport au poids total de la composition. La quantité d'huile de jojoba peut aller notamment de 35 à 90 % en poids, de préférence de 40 à 85 %, mieux de 45 à 80 % en poids par rapport au poids total de la composition.

La composition selon l'invention peut comprendre, outre l'huile de jojoba, une huile additionnelle, en particulier une huile additionnelle d'origine végétale.

Lorsque la composition comprend une huile additionnelle, l'huile additionnelle est présente en une teneur inférieure à la teneur en huile de jojoba.
En particulier, l'huile de jojoba est présente en une teneur d'au moins 50% en poids par rapport au poids total de la phase huileuse de la composition, de préférence au moins 60% en poids et mieux au moins 70% en poids, elle peut aller jusqu'à 100% en poids de la phase huileuse.

L'huile additionnelle peut être présente en une teneur allant de 0,5 à 40% en poids par rapport au poids total de la composition, de préférence de 0,5 à 30% en poids et mieux de 1 à 20% en poids.

Comme huiles utilisables dans la composition de l'invention, on peut citer par exemple :
- les huiles hydrocarbonées d'origine animale, telles que le perhydrosqualène ;
- les huiles hydrocarbonées d'origine végétale, telles que les triglycérides liquides d'acides gras comportant de 4 à 30 atomes de carbone comme les triglycérides des acides heptanoïque ou octanoïque ou encore, par exemple, les huiles de tournesol, de maïs, de soja, de courge, de pépins de raisin, de sésame, de noisette, d'abricot, de macadamia, d'arara, de coriandre, de ricin, d'avocat, les triglycérides des acides caprylique/caprique comme ceux commercialisés par la société Stearineries Dubois ou ceux commercialisés sous les dénominations Miglyol 810, 812 et 818 par la société Dynamit Nobel, l'huile de beurre de karité;
- les esters et les éthers de synthèse, notamment d'acides gras, comme les huiles de formules R¹COOR² et R¹OR² dans laquelle R¹ représente le reste d'un acide gras ou d'un alcool gras comportant de 8 à 29 atomes de carbone, et R² représente une chaîne hydrocarbonée, ramifiée ou non, contenant de 3 à 30 atomes de carbone, comme par exemple l'huile de Purcellin, le stéarate d'octyl-2-dodécyle, l'érucate d'octyl-2-dodécyle, l'isostéarate d'isostéaryle ; les esters hydroxylés comme l'isostéaryl lactate, l'octylhydroxystéarate, l'hydroxystéarate d'octyldodécyle, le diisostéaryl-malate, le citrate de triisocétyle, les heptanoates, octanoates, décanoates d'alcools gras ; les esters de polyol, comme le dioctanoate de propylène glycol, le diheptanoate de néopentylglycol et le diisononanoate de diéthylèneglycol ; et les esters du pentaérythritol comme le tétraisostéarate de pentaérythrytyle ;
- les hydrocarbures linéaires ou ramifiés, d'origine minérale ou synthétique, tels que les huiles de paraffine, volatiles ou non, et leurs dérivés, la vaseline, les polydécènes, l'isohexadecane, l'isododecane, le polyisobutène hydrogéné tel que l'huile de Parléam® ;
- les huiles de silicone comme les polyméthylsiloxanes (PDMS) volatiles ou non à chaîne siliconée linéaire ou cyclique, liquides ou pâteux à température ambiante, notamment les huiles de silicone volatiles, en particulier cyclopolydiméthylsiloxanes (cyclométhicones) telles que le cyclohexadiméthylsiloxane et le cyclopentadiméthylsiloxane ; les polydiméthyl-siloxanes comportant des groupements alkyle, alcoxy ou phényle, pendant ou en bout de chaîne siliconée, groupements ayant de 2 à 24 atomes de carbone ; les silicones phénylées comme les phényltriméthicones, les phényldiméthicones, les phényltriméthylsiloxydiphényl-siloxanes, les diphényl-diméthicones, les diphénylméthyldiphényl trisiloxanes, les 2-phényléthyltriméthylsiloxysilicates, et les polyméthylphénylsiloxanes ;
- leurs mélanges.

On peut citer en particulier les huiles choisies parmi :
- les esters issus de la réaction d'au moins un acide gras comportant au moins 6 atomes de carbone, de préférence de 6 à 26 atomes de carbone et mieux de 6 à 20 atomes de carbone, encore mieux de 6 à 16 atomes de carbone et d'au moins un alcool comprenant de 1 à 17 atomes de carbone et mieux de 3 à 15 atomes de carbone ; on peut citer notamment le myristate d'isopropyle, le palmitate d'isopropyle le caprate/caprylate d'ethyl2-hexyle (ou caprate/caprylate d'octyle), le palmitate d'éthyl-2-hexyle, le néopentanoate d'isostéaryle, l'isononanoate d'isononyle, le laurate d'hexyle, les esters d'acide lactique et d'alcools gras comprenant 12 ou 13 atomes de carbone, le carbonate de dicaprylyle tel que celui qui est commercialisé sous la dénomination CETIOL CC par la société COGNIS,
- les éthers d'acide gras comprenant de 6 à 20 atomes de carbone tel que le dicaprylyl éther (Cetiol OE de Cognis),
- les éthers de glycérol comprenant de 6 à 12 atomes de carbone comme le 2-éthyl hexyle éther de glycérol (nom INCI : ethylhexylglycerin) tel que le Sensiva SC 50 de la société Schulke & Mayr GmbH,
- les alcanes linéaires volatils, avantageusement d'origine végétale, comprenant de 7 à 17 atomes de carbone, en particulier de 9 à 15 atomes de carbone, et plus particulièrement de 11 à 13 atomes de carbone. A titre d'exemples d'alcanes linéaires volatils convenant à l'invention, on peut mentionner ceux décrits dans la demande de brevet de la société Cognis WO 2007/068371. A titre d'exemple d'alcane linéaire volatil convenant à l'invention, on peut citer le n-nonane (C₉), le n-décane (C₁₀), le n-undécane (C₁₁), le n-dodécane (C₁₂), le n-tridécane (C₁₃), le n-tétradecane (C₁₄), le n-pentadécane (C₁₅), le n-héxadécane (C₁₆) et le n-heptadécane (C₁₇) et leurs mélanges. Selon un mode de réalisation, on utilisera un mélange d'undécane (C₁₁) et de tridécane (C₁₃) comme obtenu aux exemples 1 et 2 de la demande WO2008/155059 de la Société Cognis.

On peut également citer le n-dodécane (C12) et le n-tétradécane (C14) tels que ceux vendus par Sasol respectivement sous les références PARAFOL 12-97 et PARAFOL 14-97, ainsi que leurs mélanges.

De préférence, l'huile additionnelle contient est choisie parmi les huiles végétales, les alcanes linéaires volatils, avantageusement d'origine végétale, les esters et éthers d'acide gras, et leurs mélanges.

Selon un mode de réalisation avantageux, la composition selon l'invention comprend moins de 5%, de préférence moins de 1% d'huiles minérales. De préférence, elle ne comprend que des huiles d'origine végétale.

Les compositions cosmétiques de l'invention peuvent, en outre, contenir des adjuvants habituels dans le domaine cosmétique, tels que les antioxydants, les conservateurs, les parfums, les peptisants de parfums, les matières colorantes, les charges, les actifs hydrophiles ou lipophiles. La nature des adjuvants et leurs quantités doivent être telles qu'elles ne modifient pas les propriétés de la composition selon l'invention. Les quantités de ces adjuvants sont celles classiquement utilisées dans le domaine cosmétique et par exemple de 0,001 à 10 % du poids total de la composition.

Comme actifs utilisables dans la composition de l'invention, on peut citer par exemple les agents apaisants comme l'allantoïne et le bisabolol ; les eaux florales telles que l'eau de tilleul ou l'eau de bleuet ; l'acide glycyrrhétinique et ses sels ; les antibactériens comme l'octopirox, le triclosan et le triclocarban ; les huiles essentielles ; les vitamines telles que par exemple le rétinol (vitamine A), l'acide ascorbique (vitamine C), le tocophérol (vitamine E), la niacinamide (vitamine PP ou B3), le panthénol (vitamine B5) et leurs dérivés tels que par exemple les esters de ces vitamines (palmitate, acétate, propionate), l'ascorbyl phosphate de magnésium, la vitamine C glycosylée ou acide glucopyranosyl ascorbique (Ascorbyl glucoside) ; les co-enzymes tels que le co-enzyme Q10 ou ubiquinone et le co-enzyme R ou biotine ; les hydrolysats de protéine ; les extraits végétaux et notamment les extraits de plancton ; et leurs mélanges.

Bien entendu, l'homme de l'art veillera à choisir le ou les éventuels additifs à ajouter à la composition selon l'invention de manière telle que les propriétés avantageuses attachées intrinsèquement à la composition conforme à l'invention ne soient pas, ou substantiellement pas, altérées par l'addition envisagée.
Comme charges, on peut citer les charges minérales telles que le talc ou silicate de magnésium (granulométrie: 5 microns) commercialisé sous la dénomination LUZENAC 15 M00® par la société LUZENAC, le kaolin ou silicate d'aluminium comme par exemple celui commercialisé sous la dénomination KAOLIN SUPREME® par la société IMERYS, ou les charges organiques telles que l'amidon comme par exemple le produit commercialisé sous la dénomination AMIDON DE MAIS B ® par la société ROQUETTE, les microsphères de Nylon comme celles commercialisées sous la dénomination ORGASOL 2002 UD NAT COS® par la société ATOCHEM, les microsphères à base de copolymère de chlorure de vinylidene/Acrylonitrile/methacrylonitrile enfermant de l'isobutane, expansées comme celles commercialisées sous la dénomination EXPANCEL 551 DE® par la société EXPANCEL. On peut ajouter aussi à la composition de l'invention des fibres comme par exemple des fibres de nylon (POLYAMIDE 0.9 DTEX 0.3 MM commercialisé par les Etablissements PAUL BONTE, des fibres de cellulose ou « Rayonne » (RAYON FLOCK RCISE NOOO3 MO4® commercialisé par la société CLAREMONT FLOCK CORPORATION).

Selon un mode particulier de réalisation de l'invention, la composition selon l'invention contient comme charges, des particules exfoliantes qui vont permettre le gommage de la peau. Comme particules exfoliantes, on peut utiliser des particules exfoliantes ou gommantes d'origines minérale, végétale ou organique. Ainsi, on peut utiliser par exemple les billes ou la poudre de polyéthylène, comme celles commercialisées sous la dénomination Microthene MN 727 ou Microthene MN 710-20 par la société Equistar ou comme la poudre commercialisée sous la dénomination Gotalene 120 Incolore 2 par la société Dupont ; les particules de Nylon comme celles commercialisées par la société Arkema sous la dénomination Orgasol 2002 EXD NAT COS ; les fibres comme les fibres de polyamide, comme celles commercialisées par la société Utexbel sous la dénomination PULPE POLYAMIDE 12185 TAILLE 0,3 MM ; la poudre de polychlorure de vinyle ; la pierre ponce (nom INCI : pumice) comme le ponce 3/B d'Eyraud ; les coques de noyaux de fruits broyées comme les broyats de noyaux d'abricots ou de coques de noix ; la sciure de bois ; les billes de verre ; l'alumine (oxyde d'aluminium) (nom INCI : Alumina) comme le produit commercialisé sous la dénomination Dermagrain 900 par la société Marketech International, ;les cristaux de sucre ; des billes qui fondent lors de l'application sur la peau, telles que par exemple, les sphères à base de mannitol et cellulose commercialisées sous les dénominations Unisphères par la société Induchem, les capsules à base d'agar commercialisées sous les dénominations Primasponge par la société Cognis, et les sphères à base d'esters de jojoba commercialisées sous les dénominations Florasphères par la société Floratech ; et leurs mélanges.

Les compositions selon l'invention peuvent constituer notamment des produits de nettoyage ou de démaquillage des matières kératiniques telles la peau (corps, visage, yeux, cuir chevelu) et/ou des fibres kétatiniques.

Un autre objet de l'invention est un procédé de nettoyage ou de démaquillage des matières kératiniques telles que la peau, y compris du cuir chevelu, des fibres kératiniques telles que les cils, les cheveux, et/ou des lèvres, caractérisé par le fait qu'on applique sur lesdites matières kératiniques, une composition cosmétique telle que définie ci-dessus.

Un autre objet de l'invention consiste en l'utilisation cosmétique de la composition telle que définie ci-dessus, comme produits de nettoyage et/ou de démaquillage des matières kératiniques.

Un autre objet de l'invention consiste en un procédé cosmétique de nettoyage des matières kératiniques, caractérisé par le fait qu'on applique la composition de l'invention, sur matières kératiniques, en présence d'eau, et qu'on élimine le dépôt formé et les résidus de salissure par rinçage à l'eau.

Dans le cas du nettoyage du visage, la composition selon l'invention peut constituer un masque qui est rincé après un temps de pose de 1 à 3 minutes.

Les exemples suivants sont donnés à titre d'illustration de l'invention et n'ont pas de caractère limitatif. Toutes les quantités sont données en pourcentage en poids par rapport au poids total de la composition. Les noms des composés sont indiqués selon les cas en noms chimiques ou en noms INCI.

### Exemples 1 à 4

| | **Ex 1 *hors invention*** | **Ex 2 *Invention*** | **Ex 3 *Invention*** | **Ex 4 *Invention*** |
|---|---|---|---|---|
| *Phase A* | | | | |
| Sucrose Palmitate (Surfhope SE COSME C-1616 de Mitsubishi Kagaku Foods Corp.) | 3 | 3 | 3 | 3 |
| Sucrose Laurate (Surfhope SE COSME C-1216 de Mitsubishi Kagaku Foods Corp). | 3 | 3 | 3 | 3 |
| Glycerine | 10 | 10 | 10 | 10 |
| Eau | 5 | 5 | 5 | 5 |
| Potassium sorbate | 0,3 | 0,3 | 0,3 | 0,3 |
| *Phase B* | | | | |
| Huile de Jojoba biologique (de Fytosan) | 30 | 46 | 62 | 78 |
| Huile d'Olive | 48 | 32 | 16 | - |
| *Phase C* | | | | |
| Citric acid | qsp pH 5,3 +/- 0,3 | qsp pH 5,3 +/- 0,3 | qsp pH 5,3 +/- 0,3 | qsp pH 5,3 +/- 0,3 |
| Eau | qsp 100 | qsp 100 | qsp 100 | qsp 100 |

### Mode opératoire:

On mélange les esters de sucrose et la glycérine, puis on ajoute les autres composés de la phase A et on chauffe l'ensemble à 75 - 80°C.

On mélange et on chauffe les composés de la phase B à 75 - 80°C puis on ajoute la phase B à la phase A sous faible agitation et on laisse refroidir tout en maintenant l'agitation. On ajoute la phase C lorsque le mélange est revenu à 35 - 40°C.

### Evaluation de l'efficacité démaquillante in Vivo :

On évalue l'efficacité démaquillante de chaque composition des exemples 1 à 4 sur 5 personnes selon le protocole suivant :
Sur chaque personne, 4 zones de 3x3 cm sont délimitées sur l'avant bras et 0,05 g de fond de teint Infaillible 300 (l'Oréal Paris) est appliqué au doigt sur chaque surface de 3x3 cm, puis est laissé sécher pendant 30 minutes.

0,3 g de chaque composition est appliqué sur une zone au doigt pendant 10 secondes, puis l'avant bras est ensuite passé sous un filet d'eau à température et débit fixés.

Une fois les mélanges émulsionnés et rincés, chaque modèle évalue et classe les 4 compositions testées selon leur niveau d'efficacité démaquillante sur une échelle en 4 points.

### Résultats :

Sur la moyenne des 5 modèles, l'ordre d'efficacité démaquillante est le suivant :
Composition de l'exemple 4 > Composition de l'exemple 3 > Composition de l'exemple 2 > Composition de l'exemple 1.

Ces exemples montrent bien que l'huile de jojoba présente de bonnes propriétés démaquillantes, en particulier supérieures à d'autres huiles végétales telles que l'huile d'olive.

## Revendications

1. Composition cosmétique comprenant :
- une phase aqueuse comprenant éventuellement un ou plusieurs polyols, la quantité de polyol(s) allant alors de 0,5 à 15 % en poids par rapport au poids total de la composition,
- au moins 35 % en poids d'huile de jojoba par rapport au poids total de la composition, et
- au moins un ester de sucrose et d'acide gras.

2. Composition selon la revendication 1, **caractérisée en ce que** l'ester de sucrose et d'acide gras est choisi parmi les esters issus de la réaction de sucrose(s) et d'acide(s) gras comprenant de 10 à 24 atomes de carbone, de préférence de 12 à 20 atomes de carbone, mieux de 12 à 18 atomes de carbone et encore mieux de 12 à 16 atomes de carbone.

3. Composition selon la revendication 1 ou 2, **caractérisée en ce que** l'acide gras est choisi parmi l'acide oléique, l'acide laurique, l'acide palmitique, l'acide myristique, l'acide stéarique, l'acide linoléique, l'acide caprique ou leurs mélanges.

4. Composition selon l'une quelconque des revendications précédentes, **caractérisée en ce que** l'ester de sucrose et d'acide gras est choisi parmi les esters issus de la réaction de sucrose et d'acide gras comprenant de 12 à 18 atomes de carbone, de préférence de 12 à 16 atomes de carbone.

5. Composition selon l'une quelconque des revendications précédentes, **caractérisée en ce que** l'ester de sucrose et d'acide gras est choisi parmi le sucrose laurate, le sucrose palmitate ou leur mélange.

6. Composition selon l'une quelconque des revendications précédentes, **caractérisée en ce que** l'ester de sucrose et d'acide gras est choisi parmi les monoesters, diesters, triesters de sucrose et d'acide gras ou un mélange.

7. Composition selon l'une quelconque des revendications précédentes, **caractérisée en ce que** la quantité d'ester(s) de sucrose et d'acide gras va de 0,1 à 20 % en poids, de préférence de 0,5 à 15 % en poids, mieux de 1 à 15 % en poids, encore mieux de 2 à 10% en poids par rapport au poids total de la composition.

8. Composition selon l'une quelconque des revendications précédentes, **caractérisée en ce que** le ou les esters de sucrose et d'acide gras constituent le système tensioactif principal de la composition.

9. Composition selon l'une quelconque des revendications précédentes, **caractérisée en ce que** le ou les esters de sucrose et d'acide gras constituent l'unique système tensioactif de la composition.

10. Composition selon l'une quelconque des revendications précédentes, **caractérisée en ce qu'**elle comprend de l'eau en une teneur inférieure ou égale à 20 % en poids par rapport au poids total de la composition, de préférence inférieure ou égale à 15% en poids et mieux inférieure ou égale à 10% en poids.

11. Composition selon l'une quelconque des revendications précédentes, **caractérisée en ce que** l'huile de jojoba est présente en une teneur supérieure ou égale à 40 % en poids par rapport au poids total de la composition, de préférence supérieure ou égale à 50 % en poids, mieux supérieure ou égale à 60 % en poids et encore mieux supérieure ou égale 70 % en poids par rapport au poids total de la composition.

12. Composition selon l'une quelconque des revendications précédentes, **caractérisée en ce que** la quantité d'huile de jojoba va de 35 à 90 % en poids, de préférence de 40 à 85 %, mieux de 45 à 80 % en poids par rapport au poids total de la composition.

13. Composition selon l'une quelconque des revendications précédentes, **caractérisée en ce que** l'huile de jojoba est présente en une teneur d'au moins 50% en poids par rapport au poids total de la phase huileuse de la composition, de préférence au moins 60% en poids et mieux au moins 70% en poids, pouvant aller jusqu'à 100% en poids de la phase huileuse.

14. Composition selon l'une quelconque des revendications précédentes, **caractérisée en ce qu'**elle comprend moins de 5%, de préférence moins de 1% d'huiles minérales

15. Utilisation cosmétique de la composition selon l'une quelconque des revendications 1 à 14, pour le démaquillage de la peau et/ou de la zone oculaire et/ou des lèvres.

16. Procédé de nettoyage ou de démaquillage des matières kératiniques, **caractérisé par le fait qu'**on applique sur lesdites matières kératiniques, une composition cosmétique selon l'une des revendications 1 à 14.

## Patentansprüche

1. Kosmetische Zusammensetzung, umfassend:
- eine wässrige Phase, die gegebenenfalls ein oder mehrere Polyole umfasst, wobei die Menge an Polyol(en) dann 0,5 bis 15 Gew.-%, bezogen auf das Gesamtgewicht der Zusammensetzung, beträgt,
- mindestens 35 Gew.-% Jojobaöl, bezogen auf das Gesamtgewicht der Zusammensetzung, und
- mindestens einen Saccharose-Fettsäureester.

2. Zusammensetzung nach Anspruch 1, **dadurch gekennzeichnet, dass** der Saccharose-Fettsäureester aus den Estern, die aus der Umsetzung von Saccharose(n) und Fettsäure(n) mit 10 bis 24 Kohlenstoffatomen, vorzugsweise 12 bis 20 Kohlenstoffatomen, besser 12 bis 18 Kohlenstoffatomen und noch besser 12 bis 16 Kohlenstoffatomen stammen, ausgewählt ist.

3. Zusammensetzung nach Anspruch 1 oder 2, **dadurch gekennzeichnet, dass** die Fettsäure aus Ölsäure, Laurinsäure, Palmitinsäure, Myristinsäure, Stearinsäure, Linolsäure, Caprinsäure oder Mischungen davon ausgewählt ist.

4. Zusammensetzung nach einem der vorhergehenden Ansprüche, **dadurch gekennzeichnet, dass** der Saccharose-Fettsäureester aus den Estern, die aus der Umsetzung von Saccharose und Fettsäure mit 12 bis 18 Kohlenstoffatomen und vorzugsweise 12 bis 16 Kohlenstoffatomen stammen, ausgewählt ist.

5. Zusammensetzung nach einem der vorhergehenden Ansprüche, **dadurch gekennzeichnet, dass** der Saccharose-Fettsäureester aus Saccharoselaurat, Saccharosepalmitat oder einer Mischung davon ausgewählt ist.

6. Zusammensetzung nach einem der vorhergehenden Ansprüche, **dadurch gekennzeichnet, dass** der Saccharose-Fettsäureester aus Monoestern, Diestern und Triestern von Saccharose und Fettsäure oder einer Mischung ausgewählt ist.

7. Zusammensetzung nach einem der vorhergehenden Ansprüche, **dadurch gekennzeichnet, dass** die Menge an Saccharose-Fettsäureester(n) 0,1 bis 20 Gew.-%, vorzugsweise 0,5 bis 15 Gew.-%, besser 1 bis 15 Gew.-% und noch besser 2 bis 10 Gew.-%, bezogen auf das Gesamtgewicht der Zusammensetzung, beträgt.

8. Zusammensetzung nach einem der vorhergehenden Ansprüche, **dadurch gekennzeichnet, dass** der Saccharose-Fettsäureester bzw. die Saccharose-Fettsäureester das hauptsächliche Tensidsystem der Zusammensetzung bilden.

9. Zusammensetzung nach einem der vorhergehenden Ansprüche, **dadurch gekennzeichnet, dass** der Saccharose-Fettsäureester bzw. die Saccharose-Fettsäureester das einzige Tensidsystem der Zusammensetzung bilden.

10. Zusammensetzung nach einem der vorhergehenden Ansprüche, **dadurch gekennzeichnet, dass** sie Wasser in einem Anteil kleiner gleich 20 Gew.-%, bezogen auf das Gesamtgewicht der Zusammensetzung, vorzugweise kleiner gleich 15 Gew.-% und besser kleiner gleich 10 Gew.-% umfasst.

11. Zusammensetzung nach einem der vorhergehenden Ansprüche, **dadurch gekennzeichnet, dass** das Jojobaöl in einem Anteil größer gleich 40 Gew.-%, bezogen auf das Gesamtgewicht der Zusammensetzung, vorzugsweise größer gleich 50 Gew.-%, besser größer gleich 60 Gew.-% und noch besser größer gleich 70 Gew.-%, bezogen auf das Gesamtgewicht der Zusammensetzung, vorliegt.

12. Zusammensetzung nach einem der vorhergehenden Ansprüche, **dadurch gekennzeichnet, dass** die Menge an Jojobaöl 35 bis 90 Gew.-%, vorzugsweise 40 bis 85 Gew.-% und besser 45 bis 80 Gew.-%, bezogen auf das Gesamtgewicht der Zusammensetzung, beträgt.

13. Zusammensetzung nach einem der vorhergehenden Ansprüche, **dadurch gekennzeichnet, dass** das Jojobaöl in einem Anteil von weniger als 50 Gew.-%, bezogen auf das Gesamtgewicht der Ölphase der Zusammensetzung, vorzugsweise weniger als 60 Gew.-% und besser weniger als 70 Gew.-% vorliegt und bis zu 100 Gew.-% der Ölphase betragen kann.

14. Zusammensetzung nach einem der vorhergehenden Ansprüche, **dadurch gekennzeichnet, dass** sie weniger als 5% und vorzugsweise weniger als 1% Mineralöle umfasst.

15. Kosmetische Verwendung der Zusammensetzung nach einem der Ansprüche 1 bis 14 zum Abschminken der Haut und/oder des Augenbereichs und/oder der Lippen.

16. Verfahren zum Reinigen oder Abschminken von Keratinmaterialien, **dadurch gekennzeichnet, dass** man auf die Keratinmaterialien eine kosmetische Zusammensetzung nach einem der Ansprüche 1 bis 14 aufbringt.

## Claims

1. Cosmetic composition comprising:
- an aqueous phase optionally comprising one or more polyols, the amount of polyol(s) then ranging from 0.5 to 15% by weight relative to the total weight of the composition,
- at least 35% by weight of jojoba oil relative to the total weight of the composition, and
- at least one sucrose fatty acid ester.

2. Composition according to Claim 1, **characterized in that** the sucrose fatty acid ester is chosen from the esters derived from the reaction of sucrose(s) and fatty acid(s) comprising from 10 to 24 carbon atoms, preferably from 12 to 20 carbon atoms, better still from 12 to 18 carbon atoms and even better still from 12 to 16 carbon atoms.

3. Composition according to Claim 1 or 2, **characterized in that** the fatty acid is chosen from oleic acid, lauric acid, palmitic acid, myristic acid, stearic acid, linoleic acid, capric acid or mixtures thereof.

4. Composition according to any one of the preceding claims, **characterized in that** the sucrose fatty acid ester is chosen from the esters derived from the reaction of sucrose and of fatty acid comprising from 12 to 18 carbon atoms, preferably from 12 to 16 carbon atoms.

5. Composition according to any one of the preceding claims, **characterized in that** the sucrose fatty acid ester is chosen from sucrose laurate, sucrose palmitate or a mixture thereof.

6. Composition according to any one of the preceding claims, **characterized in that** the sucrose fatty acid ester is chosen from sucrose fatty acid monoesters, diesters or triesters, or a mixture thereof.

7. Composition according to any one of the preceding claims, **characterized in that** the amount of sucrose fatty acid ester (s) ranges from 0.1 to 20% by weight, preferably from 0.5 to 15% by weight, better still from 1 to 15% by weight, even better still from 2 to 10% by weight relative to the total weight of the composition.

8. Composition according to any one of the preceding claims, **characterized in that** the sucrose fatty acid ester or esters constitute the main surfactant system of the composition.

9. Composition according to any one of the preceding claims, **characterized in that** the sucrose fatty acid ester or esters constitute the only surfactant system of the composition.

10. Composition according to any one of the preceding claims, **characterized in that** it comprises water in an amount less than or equal to 20% by weight relative to the total weight of the composition, preferably less than or equal to 15% by weight and better still less than or equal to 10% by weight.

11. Composition according to any one of the preceding claims, **characterized in that** the jojoba oil is present in an amount greater than or equal to 40% by weight relative to the total weight of the composition, preferably greater than or equal to 50% by weight, better still greater than or equal to 60% by weight and even better still greater than or equal to 70% by weight relative to the total weight of the composition.

12. Composition according to any one of the preceding claims, **characterized in that** the amount of jojoba oil ranges from 35 to 90% by weight, preferably from 40 to 85%, better still from 45 to 80% by weight relative to the total weight of the composition.

13. Composition according to any one of the preceding claims, **characterized in that** the jojoba oil is present in an amount of at least 50% by weight relative to the total weight of the oily phase of the composition, preferably at least 60% by weight and better still at least 70% by weight, possibly ranging up to 100% by weight of the oily phase.

14. Composition according to any one of the preceding claims, **characterized in that** it comprises less than 5%, preferably less than 1%, of mineral oils.

15. Cosmetic use of the composition according to any one of Claims 1 to 14, for removing makeup from the skin and/or the eye area and/or the lips.

16. Method for cleansing or for removing makeup from keratin materials, **characterized in that** a cosmetic composition according to one of Claims 1 to 14 is applied to said keratin materials.
